# EUROPEAN PATENT SPECIFICATION

(11) **EP 3 664 775 B1**
(45) Date of publication and mention of the grant of the patent: **17.03.2021**
(21) Application number: 18737620.7
(22) Date of filing: 09.07.2018
(51) Int. Cl.: A61K 8/49, A61Q 19/02, A61K 8/67

(54) **A PERSONAL CARE COMPOSITION**
HAUTAUFHELLUNG KÖRPERPFLEGEZUSAMMENSETZUNG
COMPOSITION DE SOINS PERSONNELS POUR ECLAIRCIRE LA PEAU

(30) Priority: 07.08.2017 EP 17185152
(43) Date of publication of application: 17.06.2020
(73) Proprietor: Unilever N.V., 3013 AL Rotterdam (NL); Unilever PLC, London, Greater London EC4Y 0DY (GB)
(72) Inventor: DUTTA, Maitreyee, Bangalore 560066 (IN); NAIR, Nirmala, Santosh, Bangalore 560066 (IN); RAJKUMAR, Savitha, Bangalore 560066 (IN)
(74) Representative: James, Helen Sarah
(86) International application number: PCT/EP2018/068480
(87) International publication number: WO 2019/029922

(56) References cited:
- WO-A1-2012/017734
- US-A- 4 096 240
- US-A- 5 411 741

## Description

### Field of the invention

The present invention relates to a personal care composition. In particular, the present invention relates to a personal care composition that delivers skin lightening.

### Background of the invention

People generally tend to take good care of their external surfaces that include skin, scalp, hair and oral cavity. For this, they tend to use various personal care compositions which are applied to a particular place on the human body. Often, these compositions are applied on to the skin. Personal care compositions are available in various forms like e.g. gels, emulsions, lotions, creams and ointment, and are widely used by consumers to obtain various kinds of benefits. For example, personal care compositions are used to obtain benefits like skin lightening, moisturizing and anti-aging, like e.g. preventing or minimizing sagging and wrinkling of the skin.

Skin, the outermost protective covering of living beings, is more susceptible to get affected by exposure to factors like e.g. sunlight, heat, humidity, pollution and dust. Overexposure to these factors may lead to conditions like tanning, blotchy skin, hyperpigmentation, freckles, melasma, which may in turn lead to e.g. less preferred uneven skin tones.

One of the possible ways to reduce such conditions is by ensuring minimal exposure to the affecting factors mentioned above, particularly sunlight. However, ensuring minimal exposure to such factors alone is not always sufficient and in most cases, exposure to such factors and particularly to sunlight, is unavoidable.

It is for these reasons, people have been relying on use of personal care compositions that deliver benefits like lightened skin and/or even skin tones and anti-aging. Several personal care compositions have been described which employ some or the other actives or combination of actives to deliver said benefits. For example, US4096240 (Unilever) discloses niacinamide to be a skin lightening agent.

EP 2 092 931 (Shiseido) discloses an oil-in-water milky skin cosmetic composition that in a single product delivers the feeling of use of a skin lotion and that of a skin milk. The composition has excellent stability despite high water content and low viscosity. A specific copolymer, an alkyl modified carboxyvinyl polymer and a specific alkylene oxide derivative are combined so as to obtain skin lotion of the disclosed invention.

EP 1 941 861 (Shiseido) discloses N-benzoyl-β-alanine and other specific β-alanine derivatives including 1-Piperidinepropinoic (1PP) acid, as wrinkle improving agent. These wrinkle improving agents are disclosed to be very safe for use on the skin. In particular, the application discloses wrinkle improving agent comprising as an active component one, two or more compounds selected from β-alanine derivatives represented by general formulae provided in the application.

In addition, actives like vitamin C are reported to deliver several benefits that include its use as an antioxidant, an anti-inflammatory agent and depigmentation agent (Telang 2013, Indian Dermatol Online J 4(2): 143-146). Hydroquinone and picolinamide are reported to be effective in treating melasma (Mohammad et al, 2014, Biosciences Biotechnology Research Asia 11(2): 1047-1050).

Despite efforts thus far, personal care compositions and methods for addressing consumer needs remain an area of continued interest. Consumers are always on the lookout for new and newer technologies that would meet their continued demand of obtaining benefits like skin lightening and/or even skin tones. Need therefore exists to provide an active or a combination of actives that would provide skin lightening and/or even skin tones.

It has now been found that 1PP in combination with pyridinecarboxamide, provides skin lightening. Moreover, it provides skin lightening in a synergistic way.

### Summary of the invention

In a first aspect, the present invention relates to a personal care composition comprising:
a. 1-piperidinepropionic acid; and
b. pyridinecarboxamide.

In a second aspect, the present invention relates to a method of lightening the human skin, where the method comprises the step of applying onto the skin a personal care composition according to the present invention.

In a third aspect, the present invention relates to use of a personal care composition according to the present invention for skin lightening.

### Detailed description of the invention

Unless specified otherwise, amounts as used herein are expressed in percentage by weight based on total weight of the composition and is abbreviated as "wt%".

The use of any and all examples or exemplary language e.g. "such as" provided herein is intended merely to better illuminate the invention and does not in any way limit the scope of the invention otherwise claimed.

The present invention is now described in detail. The invention relates to a personal care composition comprising 1PP and pyridinecarboxamide. The personal care composition delivers synergistic skin lightening.

### 1-Piperidinepropionic acid (1PP)

1PP (CAS number: 26371-07-3) is a compound known to deliver anti-aging benefits. In particular, it has been known to deliver anti-wrinkle benefits. The chemical structure of 1PP is as follows:

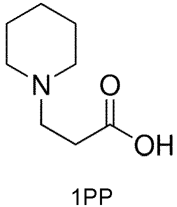

The composition comprises from 0.0001 to 10 wt%, preferably from 0.0005 to 9 wt%, more preferably from 0.001 to 8 wt%, even more preferably from 0.005 to 7 wt%, further more preferably from 0.001 wt to 6 wt%, still more preferably from 0.01 to 5 wt%, yet more preferably from 0.05 to 4 wt% and most preferably from 0.1 to 3 wt%, of 1PP.

### Pyridinecarboxamide

Examples of suitable pyridinecarboxamide compounds that may be used in the composition include 2-pyridinecarboxamide (picolinamide), 3-pyridinecarboxamide (niacinamide), 4-pyridinecarboxamide (isonicotinamide) and mixtures thereof. A preferred pyridinecarboxamide compound is any one of 2-pyridinecarboxamide, 3-pyridinecarboxamide and 4-pyridinecarboxamide. More preferred pyridinecarboxamide is 3-pyridinecarboxamide.

### 2-pyridinecarboxamide

2-pyridinecarboxaminde (CAS number: 1452-77-3), also known as picolinamide, has the following chemical structure:

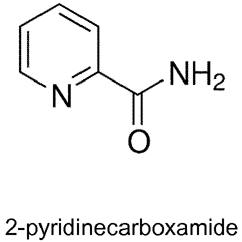

### 3-pyridinecarboxamide

*3-pyridinecarboxamide* (CAS number: 98-92-0), also known as niacinamide or nicotinamide, is an amide form of nicotinic acid. It is a vitamin found in food and also used as a dietary supplement. The chemical structure of 3-pyridinecarboxamide is as follows:

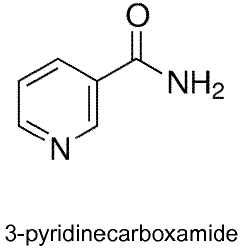

### 4-pyridinecarboxamide

4-pyridinecarboxamide (CAS number: 1453-82-3) also known as isonicotinamide and has the following chemical structure:

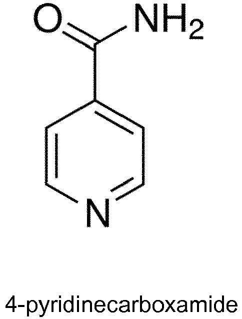

The composition comprises from 0.001 to 10 wt%, preferably from 0.005 to 8 wt%, more preferably from 0.01 to 6 wt%, even more preferably from 0.05 to 5 wt%, further more preferably from 0.1 wt to 4 wt%, still more preferably from 0.5 to 3 wt% and yet more preferably from 1 to 1.5 wt%, of pyridinecarboxamide.

### Molar ratio of 1PP to pyridinecarboxamide

Preferably, the composition comprises 1PP and pyridinecarboxamide in a molar ratio that ranges from 1:200 to 200:1, preferably from 1:150 to 150:1, more preferably from 1:100 to 100:1, even more preferably from 1:50 to 50:1, further more preferably from 1:25 to 25:1, most preferably from 1:10 to 10:1.

For example, when 1PP and 3-pyridinecarboxamide are present in a molar ratio 1:100 to 100:1, 1PP may be present from 1 part to 100 parts and at the same time, 3-pyridinecarboxamide may be present from 100 parts to 1 part. So for example, the ratio of 1PP to 3-pyridinecarboxamide may be 40:60, may be 30:90 or may be 20:80.

If more than one pyridinecarboxamide compounds are present along with 1PP, then the ratio is to be construed accordingly. For example, if 3-pyridinecarboxamide and 2-pyridinecaboxamide are present at the same time along with 1PP, the sum of amounts of 3-pyridinecarboxamide and 2-pyridinecarboxamide to be taken to construe the ratio with 1PP.

For example, if 2-pyridinecarboxamide is present in 10 parts, 3-pyridinecarboxamide is present in 10 parts and 1PP is present in 10 parts, then the molar ratio of 1PP to pyridinecarboxamide is 10:20.

### Cosmetically acceptable base

The composition may further comprise a cosmetically acceptable base. The cosmetically acceptable base acts as a diluent, dispersant or a carrier for other materials present in the composition and facilitates their distribution when the composition is applied to the skin.

The cosmetically acceptable base may be made up of ingredients that include fatty acids having from 10 to 30 carbon atoms and salts thereof, water, liquid or solid emollients, solvents, humectants, thickeners, powders. These ingredients may be used alone or as mixtures thereof to form the cosmetically acceptable base. In addition, the cosmetically acceptable base may contain skin penetration enhancers like dimethyl sulfoxide.

Examples of fatty acids that may be used in the cosmetically acceptable base include fatty acids having from 10 to 30 carbon atoms include pelargonic, lauric, myristic, palmitic, stearic, isostearic, oleic, linoleic, arachidic, behenic or erucic acid and mixtures thereof. An example of salts of fatty acid include potassium stearate.

Examples of emollients that may be used in the cosmetically acceptable base include stearyl alcohol, glyceryl monoricinoleate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as dimethylpolysiloxane, din-butyl sebacate, isopropyl myristate, isopropyl palmitate, isopropyl stearate, butyl stearate, polyethylene glycol, triethylene glycol, lanolin, cocoa butter, corn oil, cotton seed oil, olive oil, palm kernel oil, rape seed oil, safflower seed oil, evening primrose oil, soybean oil, sunflower seed oil, avocado oil, sesame seed oil, coconut oil, arachis oil, castor oil, acetylated lanolin alcohols, petroleum jelly, mineral oil, butyl myristate, isostearic acid, palmitic acid, isopropyl linoleate, lauryl lactate, myristyl lactate, decyl oleate, myristyl myristate and mixtures thereof.

Examples of solvents that may be used in the cosmetically acceptable base include ethyl alcohol, isopropanol, acetone, ethylene glycol monoethyl ether, diethylene glycol monobutyl ether and diethylene glycol monoethyl ether.

Examples of powders that may be used in the cosmetically acceptable base include chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxymethyl cellulose and ethylene glycol monostearate.

Preferred bases are water, stearic acid, potassium stearate and mixtures thereof.

The composition comprises from 5 to 99.9 wt%, preferably from 10 to 95 wt%, more preferably from 15 to 90 wt%, even more preferably from 20 to 80 wt%, further more preferably from 25 to 75 wt% and still more preferably from 30 to 70 wt% of a cosmetically acceptable base.

### Additional skin lightening agents

Preferably, the composition further comprises one or more additional skin lightening agents. These additional skin lightening agents may be selected from vitamin B6, vitamin C, vitamin A, resorcinol derivatives, 12-hydroxystearic acid, glutathione precursors, galardin, adapalene, aloe extract, ammonium lactate, arbutin, azelaic acid, butyl hydroxy anisole, butyl hydroxy toluene, citrate esters, deoxyarbutin, 1,3-diphenyl propane derivatives, 2,5-dihydroxybenzoic acid and its derivatives, 2-(4-acetoxyphenyl)-1,3-dithiane, 2-(4-hydroxyphenyl)-1,3-dithiane, ellagic acid, gluco pyranosyl-1-ascorbate, gluconic acid, glycolic acid, green tea extract, 4-Hydroxy-5-methyl-3[2H]-furanone, 4-hydroxyanisole and its derivatives, 4-hydroxybenzoic acid derivatives, hydroxycaprylic acid, inositol ascorbate, lactic acid, lemon extract, linoleic acid, magnesium ascorbyl phosphate, 5-octanoyl salicylic acid, salicylic acid, 3,4,5-trihydroxybenzyl derivatives, octadecenedioic acid, acetylglucosamine, pitera extract, symwhite, calcium pantothenate (Melano-block), seppiwhite, soybean extract (bowman birk inhibitor) and mixtures thereof.

Preferred agents that may be used as additional skin lightening agents in the composition are vitamin B6, resorcinol derivatives like e.g. 2,4-substituted resorcinol derivatives and 3,5-substituted resorcinol derivatives, hexylresorcinol and phenylethyl resorcinol, 12-hydroxystearic acid, glutathione precursors, galardin and mixtures thereof.

When incorporated in the composition, the additional skin lightening agents may be added in an amount from 0.001 to 15 wt%, preferably from 0.01 to 10 wt%, more preferably from 0.1 to 5 wt%, even more preferably from 0.5 to 3 wt%.

### Sunscreens

Preferably, the composition further comprises one or more sunscreens selected from organic sunscreens, inorganic sunscreens and mixtures thereof. Sunscreens aid in protecting the skin against ultraviolet rays in sunlight that are generally said to give rise to tanning effect.

### Organic Sunscreens

Preferably, the composition comprises one or more organic sunscreens selected from, 2-hydroxy-4-methoxybenzophenone, octyldimethyl p-aminobenzoic acid, digalloyltrioleate, 2,2-dihydroxy-4-methoxybenzophenone, ethyl-4-(bis(hydroxypropyl))aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl-p-dimethylaminobenzoate, 2-phenylbenzimidazole-5-sulfonic acid, 2-(p-dimethylaminophenyl)-5-sulfonicbenzoxazoic acid, 2-ethylhexyl-p-methoxycinnamate, dibenzoylmethane derivatives, 2-hydroxy-4- methoxybenzophenone, octyldimethyl-p-aminobenzoic acid, diethylhexyl naphthylate, Mexoryl™, Tinosorb S™, Tinosorb M™ and mixtures thereof.

Preferred dibenzoylmethane derivatives are 4-tert-butyl-4'-methoxydibenzoylmethane, 2-methyldibenzoylmethane, 4-methyl-dibenzoylethane, 4-isopropyldibenzoylmethane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane or 2,6-dimethyl-4-tert-butyl-4'-methoxydibenzoylmethane.

Preferred organic sunscreens are 2- ethylhexyl-p-methoxycinnamate (sold under the name Parsol MCX™), dibenzoylmethane derivative; in particular 4-tert-butyl-4'-methoxydibenzoylmethane (sold under the name Parsol 1789™), 2-ethylhexyl-2-cyano-3,3-diphenyl-2-propenoate (sold under the name Octocrylene™) and mixtures thereof.

When incorporated in the composition, organic sunscreens may be present in an amount from 0.1 to 15 wt%, preferably from 1 to 10 wt%, more preferably from 2 to 8 wt% and even more preferably from 3 to 6 wt%.

### Inorganic Sunscreens

The composition may further comprise inorganic sunscreens. Examples of suitable inorganic sunscreens are zinc oxide, iron oxide, silica, such as fumed silica, or titanium dioxide. Preferred inorganic sunscreens are titanium dioxide (TiO₂) and zinc oxide (ZnO).

When incorporated in the composition, inorganic sunscreens may be present in an amount from 0.1 to 15 wt%, preferably from 1 to 10 wt%, more preferably from 2 to 8 wt% and even more preferably from 2 to 5 wt%.

### Surfactants

Preferably, the composition further comprises surfactants to provide cleaning benefit. The surfactants are selected from anionic, nonionic, cationic, amphoteric surfactants and mixtures thereof.

Preferred anionic surfactants include soap, alkyl ether sulfate and sulfonates, alkyl sulfates and sulfonates, alkylbenzene sulfonates, alkyl and dialkyl sulfosuccinates, C₈-C₂₀ acyl isethionates, acyl glutamates, C₈-C₂₀ alkyl ether phosphates and combinations thereof.

Preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di- fatty acid esters of ethylene glycol; fatty acid monoglyceride; sorbitan, mono- and di- C₈-C₂₀ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic surfactants.

Examples of suitable cationic detergent surfactant include cetyl trimethylammonium bromide, benzalkonium halides that are also also known as alkyldimethylbenzylammonium halides. Preferred cationic surfactant that may be used in the composition is benzalkonium chloride, also known as alkyldimethylbenzylammonium chloride (or ADBAC).

Examples of suitable amphoteric detergent surfactant include amide, betaine and amine oxide type. Particular examples of amphoteric detergent surfactants include cocodiethanol amide and cocomonoethanol amide, cocoamidopropyl betaine and coco amido propyl amine oxide. A preferred amphoteric detergent surfactant that may be used as detergent surfactant in the composition is cocoamidopropyl betaine.

When incorporated in the composition, surfactants may be present in an amount from 0.1 to 40 wt%, preferably from 1 to 30 wt%, more preferably from 3 to 20 wt%, even more preferably from 5 to 15 wt%, further more preferably from 7 to 13 wt%.

### Optional Ingredients

The composition may further comprise a range of other optional ingredients that include antioxidants, binders, biological additives, buffering agents, colorants, polymers, astringents, fragrance, opacifying agents, conditioners, exfoliating agents, pH adjusters, preservatives, natural extracts, skin sensates, skin soothing agents, and skin healing agents.

### Product Form and Packaging

The composition is preferably formulated in the form of a powder, flake, lotion, cream, gel or mousse. The composition can be a leave-on or wash-off type of composition. The composition is preferably a leave-on type of composition. The packaging for the composition of this invention can be a patch, bottle, tube, roll-ball applicator, propellant driven aerosol device, squeeze container or lidded jar.

### Method of skin lightening

In a second aspect, the present invention further relates to a method of lightening the human skin. The method comprises the step of:
a. applying the composition according to any one of claims 1 to 13 onto the human skin; and
b. optionally, rinsing with water.

When the personal care composition comprising 1PP and pyridinecarboxamide is applied on to the skin, synergistic skin lightening is obtained.

In a third aspect, the present invention relates to use of the composition comprising 1PP and pyridinecarboxamide for obtaining skin lightening.

The invention will now be illustrated by means of the following non limiting examples.

### Examples

### Materials

(i) Neonatal primary human epidermal melanocytes, Medium 254 and Human Melanocyte Growth Supplement-2 (all from Life Technologies)
(ii) HaCaT keratinocytes (from Prof. Fusenig, Heidelberg, Germany),
(iii) EpiLife Medium and Human Keratinocyte Growth Supplement (both from Life Technologies)
(iv) 3-pyridinecarboxamide (Sigma Aldrich)
(v) 1PP (Sigma Aldrich)
(vi) Dulbecco's minimal essential medium (Sigma Aldrich)
(vii) Calcein-AM: Calcein acetoxymethyl ester (Sigma Aldrich)
(viii) Dimethyl sulfoxide (Sigma Aldrich)
(ix) Sodium hydroxide (Merck Specialities Pvt. Ltd.)
(x) Phosphate buffered saline
(xi) Foetal Bovine Serum (Gibco)

### Protocols

### Cell cultures

Neonatal primary human epidermal melanocytes were sourced from Life Technologies. Melanocytes were maintained in Medium 254 supplemented with Human Melanocyte Growth Supplement-2 (MGM).

HaCaT keratinocytes were maintained in DMEM medium containing 10% heat inactivated foetal bovine serum. All media were also supplemented with 10 U/mL penicillin G and 0.1 mg/mL streptomycin sulphate. Both cell types were maintained at 37°C in a humidified incubator with 5% CO₂ atmosphere.

Maintenance and sub-culturing of cells were carried out as per the manufacturer's instructions. For setup of co-cultures, HaCaT keratinocytes and melanocytes, each 40,000 cells were mixed (1:1 ratio) in 1 mL of a 1:1 mix of MGM:KGM (KGM: EpiLife Medium supplemented with Human Keratinocyte Growth Supplement) and seeded as such per well, in 12 well Nunc plates.

### Active addition

Actives were prepared in media and test concentrations were added to cells along with relevant controls 24 h post seeding. After 72 h of active treatment, cell viability (using calcein method) and measurement of cellular melanin content was determined as described below.

### Cell Viability assay

Briefly, spent media was removed and cells washed once with 0.4 mL of Phosphate buffered saline. Fresh 1 µM calcein- acetoxymethyl in phosphate buffered saline was added to each well. Plates were incubated for 30 min. at 37°C under 5% CO₂ incubator. Calcein fluorescence was measured (excitation at 490 nm and emission at 520 nm) using TECAN M1000 plate reader.

### Melanin content assay

After calcein fluorescence readings were obtained, cells were drained and fresh 0.15 mL of 1N NaOH (in 10% DMSO) per well was added. Cells were lysed by resuspension and incubated (60°C for 1 h). Then, 0.1 mL of this lysate was transferred to a fresh 384-well plate and OD was measured at 405nm in a TECAN M1000 plate reader (estimate of relative melanin content).

**Table 1: Average % reduction in cellular melanin content in response to different treatments**

| **Ex.** | **Treatment** | **Inhibition in Cellular Melanin (%)** | **Standard Error** |
|---|---|---|---|
| A | Media | 0 | 0 |
| B | 1PP (100uM) | 1 | 2 |
| C | 1PP (1mM) | -5 | 3 |
| D | 1PP (10mM) | 0 | 4 |
| E | 3-pyridinecarboxamide (10mM) | 0 | 3 |
| 1 | 1PP (100µM) + 3-pyridinecarboxamide (10mM) | 14 | 1 |
| 2 | 1PP (1mM) + 3-pyridinecarboxamide (10mM) | 26 | 3 |
| 3 | 1PP (10mM) + 3-pyridinecarboxamide (10mM) | 22 | 4 |

As can be seen from data in table 1, 1PP when used in combination with 3-pyridinecarboxamide, provides synergistic skin lightening.

In conclusion, 1PP in combination with pyridinecarboxamide provides synergistic skin lightening.

## Claims

1. A personal care composition comprising:
a. 1-piperidinepropionic acid; and
b. pyridinecarboxamide.

2. A personal care composition according to claim 1 wherein the molar ratio of 1-piperidinepropionic acid to pyridinecarboxamide is 1:200 to 200:1.

3. A personal care composition according to claim 1 wherein the molar ratio of 1-piperidinepropionic acid to pyridinecarboxamide is 1:100 to 100:1.

4. A personal care composition according to any one of claims 1 to 3 wherein the composition comprises 0.0001 to 10 wt% of 1-piperidinepropionic acid.

5. A personal care composition according to any one of claims 1 to 4 wherein the composition comprises 0.001 to 10 wt% of pyridinecarboxamide.

6. A personal care composition according to any one of claims 1 to 5 wherein the pyridinecarboxamide is selected from 2-pyridinecarboxamide, 3-pyridinecarboxamide, 4-pyridinecarboxamide and mixtures thereof.

7. A personal care composition according to any one of claims 1 to 6 wherein the composition further comprises a cosmetically acceptable base.

8. A personal care composition according to any one of claims 1 to 7 further comprising 0.001 to 15 wt% of additional skin lightening agent.

9. A personal care composition according to any one of claims 1 to 8 further comprising 0.1 to 15 wt% of at least one organic sunscreen.

10. A personal care composition according to any one of claims 1 to 9 further comprising 0.1 to 15 wt% of at least one inorganic sunscreen.

11. A personal care composition according to any one of claims 1 to 10 further comprising 0.1 to 40 wt% of at least one surfactant.

12. A personal care composition according to any one of claims 1 to 11, wherein the composition is a leave-on composition.

13. A personal care composition according to any one of claims 1 to 11, wherein the composition is a wash-off composition.

14. A non-therapeutic method of lightening the human skin, the method comprising:
a. applying onto the skin a personal care composition according to any one of claims 1 to 13; and
b. optionally, rinsing with water.

15. Non-therapeutic use of the composition according to any one of claims 1 to 13 for skin lightening.

## Patentansprüche

1. Körperpflegezusammensetzung, umfassend:
a. 1-Piperidinpropionsäure; und
b. Pyridincarboxamid.

2. Körperpflegezusammensetzung nach Anspruch 1, wobei das Molverhältnis von 1-Piperidinpropionsäure zu Pyridincarboxamid 1:200 bis 200:1 beträgt.

3. Körperpflegezusammensetzung nach Anspruch 1, wobei das Molverhältnis von 1-Piperidinpropionsäure zu Pyridincarboxamid 1:100 bis 100:1 beträgt.

4. Körperpflegezusammensetzung nach irgendeinem der Ansprüche 1 bis 3, wobei die Zusammensetzung 0,0001 bis 10 Gew.-% 1-Piperidinpropionsäure umfasst.

5. Körperpflegezusammensetzung nach irgendeinem der Ansprüche 1 bis 4, wobei die Zusammensetzung 0,001 bis 10 Gew.-% Pyridincarboxamid umfasst.

6. Körperpflegezusammensetzung nach irgendeinem der Ansprüche 1 bis 5, wobei das Pyridincarboxamid ausgewählt ist aus 2-Pyridincarboxamid, 3-Pyridincarboxamid, 4-Pyridincarboxamid und Mischungen davon.

7. Körperpflegezusammensetzung nach irgendeinem der Ansprüche 1 bis 6, wobei die Zusammensetzung ferner eine kosmetisch akzeptable Basis umfasst.

8. Körperpflegezusammensetzung nach irgendeinem der Ansprüche 1 bis 7, ferner umfassend 0,001 bis 15 Gew.-% zusätzliches Hautaufhellungsmittel.

9. Körperpflegezusammensetzung nach irgendeinem der Ansprüche 1 bis 8, ferner umfassend 0,1 bis 15 Gew.-% mindestens eines organischen Sonnenschutzmittels.

10. Körperpflegezusammensetzung nach irgendeinem der Ansprüche 1 bis 9, ferner umfassend 0,1 bis 15 Gew.-% mindestens eines anorganischen Sonnenschutzmittels.

11. Körperpflegezusammensetzung nach irgendeinem der Ansprüche 1 bis 10, ferner umfassend 0,1 bis 40 Gew.-% mindestens eines Tensids.

12. Körperpflegezusammensetzung nach irgendeinem der Ansprüche 1 bis 11, wobei die Zusammensetzung eine Leave-on-Zusammensetzung ist.

13. Körperpflegezusammensetzung nach irgendeinem der Ansprüche 1 bis 11, wobei die Zusammensetzung eine abwaschbare Zusammensetzung ist.

14. Nichttherapeutisches Verfahren zum Aufhellen der menschlichen Haut, wobei das Verfahren umfasst:
a. Aufbringen einer Körperpflegezusammensetzung nach irgendeinem der Ansprüche 1 bis 13 auf die Haut; und
b. gegebenenfalls Abspülen mit Wasser.

15. Nichttherapeutische Verwendung der Zusammensetzung nach irgendeinem der Ansprüche 1 bis 13 zur Hautaufhellung.

## Revendications

1. Composition pour le soin de la personne comprenant :
a. de l'acide 1-pipéridinepropionique ; et
b. du pyridinecarboxamide.

2. Composition pour le soin de la personne selon la revendication 1, dans laquelle le rapport molaire d'acide 1-pipéridinepropionique à pyridinecarboxamide est de 1:200 à 200:1.

3. Composition pour le soin de la personne selon la revendication 1, dans laquelle le rapport molaire d'acide 1-pipéridinepropionique à pyridinecarboxamide est de 1:100 à 100:1.

4. Composition pour le soin de la personne selon l'une quelconque des revendications 1 à 3, dans laquelle la composition comprend de 0,0001 à 10 % en masse d'acide 1-pipéridinepropionique.

5. Composition pour le soin de la personne selon l'une quelconque des revendications 1 à 4, dans laquelle la composition comprend de 0,001 à 10 % en masse de pyridinecarboxamide.

6. Composition pour le soin de la personne selon l'une quelconque des revendications 1 à 5, dans laquelle le pyridinecarboxamide est choisi parmi le 2-pyridinecarboxamide, 3-pyridinecarboxamide, 4-pyridinecarboxamide et des mélanges de ceux-ci.

7. Composition pour le soin de la personne selon l'une quelconque des revendications 1 à 6, dans laquelle la composition comprend de plus une base cosmétiquement acceptable.

8. Composition pour le soin de la personne selon l'une quelconque des revendications 1 à 7 comprenant de plus de 0,001 à 15 % en masse d'agent éclaircissant la peau supplémentaire.

9. Composition pour le soin de la personne selon l'une quelconque des revendications 1 à 8 comprenant de plus de 0,1 à 15 % en masse d'au moins un écran solaire organique.

10. Composition pour le soin de la personne selon l'une quelconque des revendications 1 à 9 comprenant de plus de 0,1 à 15 % en masse d'au moins un écran solaire inorganique.

11. Composition pour le soin de la personne selon l'une quelconque des revendications 1 à 10 comprenant de plus de 0,1 à 40 % en masse d'au moins un tensioactif.

12. Composition pour le soin de la personne selon l'une quelconque des revendications 1 à 11, dans laquelle la composition est une composition sans rinçage.

13. Composition pour le soin de la personne selon l'une quelconque des revendications 1 à 11, dans laquelle la composition est une composition à rincer.

14. Procédé non-thérapeutique d'éclaircissement de la peau humaine, le procédé comprenant :
a. l'application sur la peau d'une composition pour le soin de la personne selon l'une quelconque des revendications 1 à 13 ; et
b. éventuellement, le rinçage avec de l'eau.

15. Utilisation non-thérapeutique de la composition selon l'une quelconque des revendications 1 à 13 pour l'éclaircissement de la peau.
